# EUROPEAN PATENT APPLICATION

(11) **EP 4 674 427 A1**
(43) Date of publication of application: **07.01.2026**
(21) Application number: 24763265.6
(22) Date of filing: 01.03.2024
(51) Int. Cl.: A61K 38/17, A61K 48/00, C12N 15/861, A61P 3/00, A61P 35/00

(54) **METHOD FOR TREATING OR AMELIORATING CACHEXIA**

(30) Priority: 01.03.2023 CN 202310180931
(71) Applicant: Hangzhou Institute for Advanced Study, UCAS, Hangzhou, Zhejiang 310024 (CN); Suzhou Yidan Biology Co, Ltd., Suzhou, Jiangsu 215000 (CN)
(72) Inventor: LI, Lin, Shanghai 200032 (CN); DENG, Qi, Suzhou, Jiangsu 215000 (CN); DENG, Qi, China (Jiangsu) Pilot Free Trade Zone Suzhou, Jiangsu 215000 (CN)
(74) Representative: Winter, Brandl - Partnerschaft mbB
(86) International application number: PCT/CN2024/079680
(87) International publication number: WO 2024/179585

(57) **Abstract**

The present invention provides a method for treating or ameliorating cachexia. The present invention provides use of nucleobindins (NUCBs). By the method of the present invention, (a) body weight reduction and (b) body fat reduction which are caused by cachexia can be well ameliorated or treated, thereby improving the quality of life of cachexia patients.

## Description

### TECHNICAL FIELD

The present invention relates to the field of biomedicine. More specifically, the present invention relates to a method for treating or ameliorating cachexia.

### BACKGROUND

Cachexia is a complex clinical metabolic syndrome in which the body's own tissues are consumed, manifested as body weight reduction, muscle loss and/or fat loss, and decreased appetite. It can be found in a variety of diseases, including tumors, AIDS, severe trauma, post-surgery, malabsorption and severe sepsis, etc. Among them, cachexia associated with tumors is the most common, which is called tumor cachexia. Tumor cachexia is a necessary stage before the death of more than 80% of advanced cancer patients, among which the death of 20% of cancer patients are directly related to cachexia. The incidence of cachexia is also very high during post-surgery radiotherapy or chemotherapy, etc. The occurrence of cachexia mostly refers to the body being in a state of severe functional dysfunction. Patients often show phenomena of systemic functional failure such as significant weight loss, anemia, mental depression, etc., which seriously affects the treatment effect on and quality of life of the patients.

Cachexia is a serious clinical syndrome that is severely underestimated and underrecognized. Currently, neither there is any widely accepted specific treatment drug for cachexia, nor there is any specific treatment drug for cachexia as approved by FDA, USA. Its incidence varies depending on a primary disease, ranging from 5%-15% in chronic congestive heart failure and chronic obstructive pulmonary diseases to 60%-80% in advanced tumors. Currently, the purpose of treatment for cachexia, especially tumor cachexia, is to improve symptoms and enhance quality of life, rather than, and it is impossible to, reverse the disease progression.

Therefore, it is particularly urgent and important to find a novel drug or therapeutic target that can improve cachexia.

### SUMMARY

An objective of the present invention is to provide a novel approach to treat or ameliorate cachexia.

In a first aspect, the present invention provides use of nucleobindins (NUCBs), or an encoding gene or expression vector thereof in preparation of a composition or formulation for ameliorating or treating cachexia.

In another preferred embodiment, the cachexia includes tumor cachexia.

In another preferred embodiment, the tumor includes lung cancer, pancreatic cancer, gastroesophageal cancer, head and neck cancer, colorectal cancer, blood cancer, breast cancer, and prostate cancer.

In another preferred embodiment, the composition or formulation is used for increasing (a) body weight and (b) body fat in a patient with cachexia.

In another preferred embodiment, the NUCB is derived from a mammal; preferably, from human, a mouse, a rat, or a rabbit; and more preferably, from human.

In another preferred embodiment, the NUCB gene includes a wild-type NUCB gene and a mutant NUCB gene.

In another preferred embodiment, the mutant type includes a mutant form in which the function of the encoded protein is not changed after mutation (i.e., the function is identical or substantially identical to that of a wild-type encoded protein).

In another preferred embodiment, a polypeptide encoded by the mutant NUCB gene is identical or substantially identical to a polypeptide encoded by the wild-type NUCB gene.

In another preferred embodiment, the mutant NUCB gene includes a polynucleotide having a homology ≥ 80% (preferably ≥ 90%, more preferably ≥ 95%, and more preferably ≥ 98% or 99%) compared to the wild-type NUCB gene.

In another preferred embodiment, the mutant NUCB gene includes a polynucleotide having 1-60 (preferably 1-30, and more preferably 1-10) nucleotides truncated from or added to the 5' terminal and/or 3' terminal of the wild-type NUCB gene.

In another preferred embodiment, the NUCB gene includes a cDNA sequence, a genomic sequence, or a combination thereof.

In another preferred embodiment, the NUCB protein includes an active fragment of NUCB or a derivative thereof.

In another preferred embodiment, the homology between the active fragment or a derivative thereof and the NUCB is at least 90%, preferably 95%, and more preferably 98% or 99%.

In another preferred embodiment, the active fragment or a derivative thereof has at least 80%, 85%, 90%, 95%, or 100% of the activity of the NUCB.

In another preferred embodiment, the NUCB protein is selected from the group consisting of NUCB1, NUCB2 or a combination thereof.

In another preferred embodiment, an amino acid sequence of the NUCB protein is selected from the following group:
(i) a polypeptide having an amino acid sequence as shown in any one of SEQ ID NOs.: 1-4;
(ii) a polypeptide derived from (i), which has the function of the protein and is formed by substituting, deleting or adding one or several (e.g. 1-10) amino acid residues of the amino acid sequence as shown in any one of SEQ ID NOs.: 1-4; or
(iii) a polypeptide having an amino acid sequence with homology of ≥ 90% (preferably ≥ 95%, and more preferably ≥ 98% or 99%) to the amino acid sequence as shown in any one of SEQ ID NOs.: 1-4, and having the function of the protein.

In another preferred embodiment, a nucleotide sequence of the NUCB gene is selected from the following group:
(a) a polynucleotide encoding a polypeptide as shown in any one of SEQ ID NOs.: 1-4;
(b) a polynucleotide of which the sequence is as shown in any one of SEQ ID NOs.: 5-8;
(c) a polynucleotide having a nucleotide sequence with homology of ≥ 95% (preferably ≥ 98%, and more preferably ≥ 99%) to a sequence as shown in any one of SEQ ID NOs.: 5-8;
(d) a polynucleotide having 1-60 (preferably 1-30, and more preferably 1-10) nucleotides truncated from or added to the 5' terminal and/or 3' terminal of the polynucleotide as shown in any one of SEQ ID NOs.: 5-8; and
(e) a polynucleotide complementary to the polynucleotide as described in any one of (a)-(d).

In another preferred embodiment, the NUCB protein is as shown in any one of SEQ ID NOs.: 1-4.

In another preferred embodiment, the nucleic acid encoding the NUCB protein is as shown in any one of SEQ ID NOs.: 5-8.

In another preferred embodiment, the concentration of the NUCB protein, or an encoding gene or expression vector thereof is 2.5 × 10⁹ - 2.5 × 10¹⁰, preferably 5 × 10⁹ - 1.25 × 1¹⁰, and more preferably 7.5 × 10⁹ - 1 × 10¹⁰ (an AAV virus titer, administered via tail vein/mouse)

In another preferred embodiment, the expression vector includes a viral vector.

In another preferred embodiment, the viral vector is selected from the group consisting of: an adeno-associated virus (AAV), an adenovirus, a lentivirus, a retrovirus, a herpes virus, SV40, a poxvirus, or a combination thereof.

In another preferred embodiment, the vector is selected from the group consisting of: a lentivirus, an adenovirus, an adeno-associated virus (AAV), or a combination thereof, and preferably, the vector is the adeno-associated virus (AAV).

In another preferred embodiment, the vector includes AAV2/8.

In another preferred embodiment, the NUCB protein is administered by packaging into an AAV virus.

In another preferred embodiment, the composition includes a pharmaceutical composition.

In another preferred embodiment, the composition contains (a) an NUCB protein, or an encoding gene or expression vector thereof; and (b) a pharmaceutically acceptable carrier.

In another preferred embodiment, a dosage form of the composition is selected from the following group: a lyophilized formulation, a liquid formulation, or a combination thereof.

In another preferred embodiment, the dosage form of the composition is a liquid formulation.

In another preferred embodiment, the dosage form of the composition is an injection form.

In another preferred embodiment, in the composition, the component (a) accounts for 1-99 wt%, preferably 10-90 wt%, and more preferably 30-70 wt% of the total weight of the pharmaceutical composition.

In another preferred embodiment, the composition or formulation further includes other drugs for ameliorating or treating cachexia.

In another preferred embodiment, the other drugs for ameliorating or treating cachexia are selected from the following group: corticosteroid, a progesterone analog, olanzapine, anamorelin, or a combination thereof.

In another preferred embodiment, the composition or formulation can be used alone or in combination in an application for ameliorating or treating cachexia.

In another preferred embodiment, the combined use includes: combined use with other drugs for ameliorating or treating cachexia.

A second aspect of the present invention provides a pharmaceutical composition including:
(a1) a first active ingredient for ameliorating or treating cachexia, the first active ingredient including: an NUCB protein, or an encoding gene or expression vector thereof;
(a2) optionally, a second active ingredient for ameliorating or treating cachexia, the second active ingredient including: other drugs for ameliorating or treating cachexia; and
(b) a pharmaceutically acceptable carrier.

In another preferred embodiment, in the pharmaceutical composition, the component (a1) accounts for 1-99 wt%, preferably 10-90 wt%, and more preferably 30-70 wt% of the total weight of the pharmaceutical composition.

In another preferred embodiment, in the pharmaceutical composition, the component (a2) accounts for 1-99 wt%, preferably 10-90 wt%, and more preferably 30-70 wt% of the total weight of the pharmaceutical composition.

In another preferred embodiment, a weight ratio of the first active ingredient to the second active ingredient is 1:100 to 100:1, and preferably 1:10 to 10:1.

In another preferred embodiment, the other drugs for ameliorating or treating cachexia are selected from the following group: corticosteroid, a progesterone analog, olanzapine, anamorelin, or a combination thereof.

In another preferred embodiment, the pharmaceutical composition may be a single compound or a mixture of multiple compounds.

In another preferred embodiment, the pharmaceutical composition is used for preparing a drug or formulation for ameliorating or treating cachexia.

In another preferred embodiment, the pharmaceutical dosage form is an injection or injection medicine.

In another preferred embodiment, the total content of the active ingredient (a1) and the active ingredient (a2) is 1-99 wt%, and more preferably 5-90 wt% of the total weight of the composition.

A third aspect of the present invention provides a kit including:
(i) a first container, and an active ingredient located in the first container: (a1) NUCB protein, or an encoding gene or expression vector thereof, or a drug containing an active ingredient (a); and
(ii) optionally a second container, and an active ingredient located in the second container: (a2) other drugs for ameliorating or treating cachexia, or a drug containing the active ingredient (a2).

In another preferred embodiment, the first container and the second container are the same or different containers.

In another preferred embodiment, the drug in the first container is a monomer preparation containing the NUCB protein, or an encoding gene or expression vector thereof.

In another preferred embodiment, the drug in the second container is a monomer preparation containing other drugs for ameliorating or treating cachexia.

In another preferred embodiment, the dosage form of the drug is an injection form.

In another preferred embodiment, the kit further includes instructions, in which instructions for co-administering the active ingredient (a1) and the active ingredient (a2) to ameliorate or treat cachexia are recorded.

A fourth aspect of the present invention provides use of the pharmaceutical composition of the second aspect of the present invention or the kit of the third aspect of the present invention in preparation of a drug for ameliorating or treating cachexia.

A fifth aspect of the present invention provides a method for ameliorating or treating cachexia, including the steps of:
Administering an NUCB protein, or an encoding gene or expression vector thereof, the pharmaceutical composition of the second aspect of the present invention, or the kit of the third aspect of the present invention to a subject in need thereof.

In another preferred embodiment, the administration includes injection (preferably intravenous injection).

In another preferred embodiment, the subject includes human or a non-human mammal.

In another preferred embodiment, the non-human mammal includes a rodent and a primate, and preferably a mouse, a rat, a rabbit, and a monkey.

In another preferred embodiment, the expression vector of the NUCB protein is administered at a frequency of once every 3-6 months.

In another preferred embodiment, the NUCB protein, or an encoding gene or expression vector thereof is administered for a time of 1-20 weeks, preferably 2-12 weeks, and more preferably 4-8 weeks.

A sixth aspect of the present invention provides a method for screening a candidate drug for ameliorating or treating cachexia, including the steps of:
(a) in a culture system of in a test group in the presence of a test substance, culturing cells expressing an NUCB protein for a period of time T1, and detecting an expression level E1 and/or activity A1 of the NUCB protein in the culture system of the test group;
and in a control group in the absence of the test substance and with the same other conditions, detecting an expression level E2 and/or activity A2 of the NUCB protein in the culture system of the control group;
(b1) comparing E1 and E2, where if E1 is significantly higher than E2, it indicates that the test substance is a candidate drug for ameliorating or treating cachexia; and/or
(b2) comparing A1 and A2, where if A1 is significantly higher than A2, it indicates that the test substance is a candidate drug for ameliorating or treating cachexia.

In another preferred embodiment, the cells are mammalian cells.

In another preferred embodiment, the cells include tumor cells.

In another preferred embodiment, the cells are selected from the group consisting of: lung cancer cells, pancreatic cancer cells, gastroesophageal cancer cells, head and neck cancer cells, colorectal cancer cells, blood cancer cells, breast cancer cells, prostate cancer cells, or a combination thereof.

In another preferred embodiment, the cells are selected from the group consisting of: B16F10 cells, C26 cells, MAC16 cells, EHS cells, MCG101 cells, LLC cells, or a combination thereof.

In another preferred embodiment, the cells are cells cultured *in vitro.*

In another preferred embodiment, the "significantly higher than" refers to E1/E2 ≥ 2, preferably ≥ 3, and more preferably ≥ 4.

In another preferred embodiment, the "significantly lower than" refers to A1/A2 ≥ 2, preferably ≥ 3, and more preferably ≥ 4.

In another preferred embodiment, the method is non-diagnostic and non-therapeutic.

In another preferred embodiment, the method includes step (c): administering the candidate drug determined in the step (a) to a non-human mammal, thereby determining its effect on cachexia in the non-human mammal.

In another preferred embodiment, the test substance is selected from the following group: a small-molecule compound, an antibody, a polypeptide, a nucleic acid, or a combination thereof.

A seventh aspect of the present invention provides a method for screening a candidate drug for treating or ameliorating cachexia, including the steps of:
(a) in a test group in the presence of a test compound, detecting an mRNA expression level or protein level L1 of NUCB1 and/or NUCB2 genes in cells of the test group;
   and in a control group in the absence of the test compound and with the same other conditions, detecting an mRNA expression level or protein level L2 of the NUCB1 and/or NUCB2 in the control group; and
(b) comparing L1 and L2, wherein if L2 is significantly lower than L1, it indicates that the test compound is a candidate drug for treating or ameliorating cachexia.

In another preferred embodiment, the cells are cells cultured *in vitro.*

In another preferred embodiment, the cells are living tissue cells.

In another preferred embodiment, the method is non-diagnostic and non-therapeutic.

In another preferred embodiment, the method includes step (c): administering the candidate drug determined in the step (a) to a non-human mammal, and further determining its effect on treating or ameliorating cachexia in the non-human mammal.

It should be understood that, within the scope of the present invention, the aforementioned technical features of the present invention and the technical features described in detail hereafter (e.g., in examples) can be combined with each other to form a new or preferred technical solution. As limited by the length, it will not be repeated any more here.

### BRIEF DESCRIPTION OF DRAWINGS

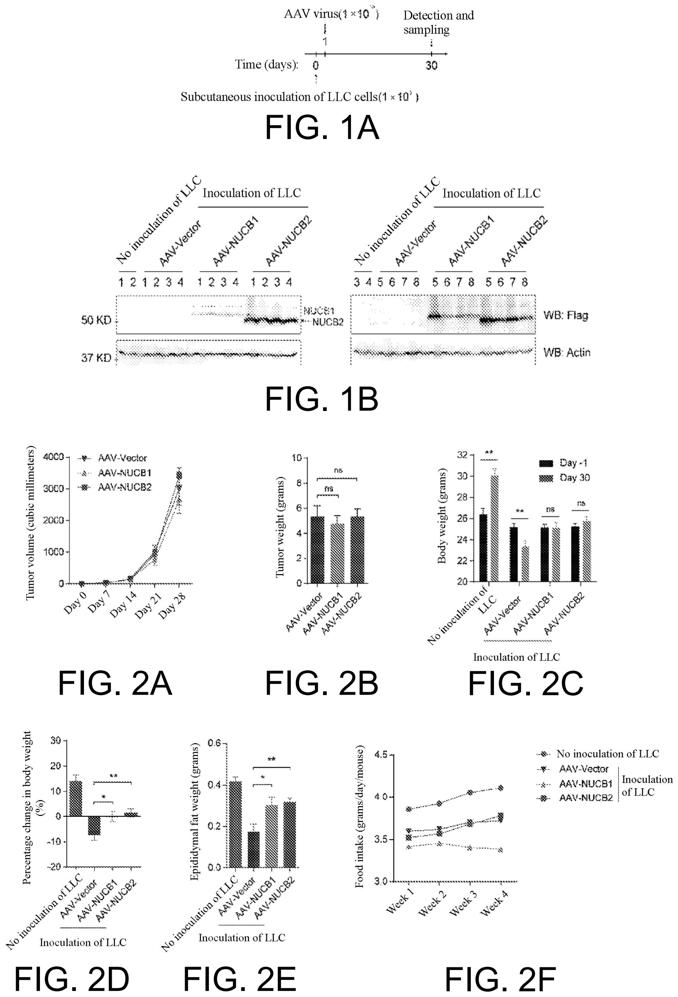

FIG. 1 shows the expression of an AAV virus in the body of a mouse. A. an experimental process; and B. an expression result of an AAV virus in the liver of the mouse.
FIG. 2 shows that both NUCB1 and NUCB2 proteins delivered via AAV significantly alleviate tumor-induced cachexia in a mouse model. A. overexpression of the AAV-delivered NUCB1 or NUCB2 does not change the growth of LLC tumors; B. overexpression of the AAV-delivered NUCB1 or NUCB2 does not change the final weight of LLC tumors; C. overexpression of the AAV-delivered NUCB1 or NUCB2 reduces LLC tumor-induced body weight reduction in the mice; D. overexpression of the AAV-delivered NUCB1 or NUCB2 reduces LLC tumor-induced body weight reduction in the mice (in percentage); E. overexpression of the AAV-delivered NUCB1 or NUCB2 reduces LLC tumor-induced body fat reduction in the mice (epididymal white fat); and F. overexpression of the AAV-delivered NUCB1 or NUCB2 does not change LLC tumor-induced food intake reduction in the mice.

### DETAILED DESCRIPTION

After long-term, extensive and in-depth research, the present inventors have discovered for the first time that nucleobindins (NUCBs) can ameliorate or treat cachexia. Furthermore, it has been demonstrated through experiments that administering NUCB proteins, such as NUCB 1 and/or NUCB2 proteins, to a cachexia mouse model can significantly ameliorate cachexia-induced (a) body weight reduction and (b) body fat reduction. On this basis, the inventors completed the present invention.

### Terminology Description

Unless defined otherwise, all technical and scientific terms used herein have the same meaning as commonly understood by those of ordinary skills in the art to which the present invention belongs.

As used herein, when used in reference to a specific recited numerical value, the term "about" means that the value may vary by no more than 1% from the recited value. For example, as used herein, the expression "about 100" includes 99 and 101 and all values therebetween (e.g., 99.1, 99.2, 99.3, 99.4, etc.).

As used herein, the terms "comprising", "including" or "containing" may be open, semi-closed, or closed. In other words, the term also includes "consisting essentially of," or "consisting of.

### Cachexia

Cachexia is a multifactorial systemic syndrome characterized by persistent skeletal muscle loss (with or without adipose tissue loss) that cannot be completely reversed by conventional nutritional support and leads to progressive functional impairment. The main manifestations are: body weight reduction, anorexia, and systemic inflammatory response.

Also, cachexia is a complex clinical metabolic syndrome in which the body's own tissues are consumed, manifested as body weight reduction, muscle loss and/or fat loss, and decreased appetite. It can be found in a variety of diseases, including tumors, AIDS, severe trauma, post-operative radiotherapy or chemotherapy, malabsorption and severe sepsis, etc. Among them, cachexia associated with tumors is the most common, which is called tumor cachexia.

In the present invention, cachexia includes tumor cachexia, e.g. lung cancer cachexia, pancreatic cancer cachexia, gastroesophageal cancer cachexia, head and neck cancer cachexia, colorectal cancer cachexia, blood cancer cachexia, breast cancer cachexia, and prostate cancer cachexia.

### NUCB protein and an encoding gene thereof

In the present invention, "nucleobindins", "NUCB proteins", "NUCB1 and NUCB2 proteins" can be used interchangeably and have the same meaning.

NUCBs (nucleobindins) proteins are a family of calcium ion-binding secretory proteins, including NUCB1 and NUCB2 proteins. The function of the NUCB2 protein was first reported in 2006 to suppress appetite and reduce body weight, but subsequent studies showed that under physiological conditions, NUCB2 had no appetite and body weight regulation function. So far, the biological function of NUCB1 is still unclear. Therefore, the biological function and molecular mechanism of the NUCBs family remain unclear. The NUCB1 and NUCB2 proteins are conserved in evolution and have high homology. For example, the similarity between human and rat NUCB2 proteins is 87.4%, and that between rat and mouse NUCB2 proteins is 95.7%.

In the present invention, the terms "NUCB gene" and "NUCB polynucleotide" are used interchangeably and refer to a nucleic acid sequence having a NUCB nucleotide sequence.

The gene full length of the human NUCB1 gene is 1,485 bp (with an accession number of NM_006184.6, please add), at the location of Gene ID: 4924.

The gene full length of the human NUCB2 gene is 1,265 bp (with an accession number of NM_001352661.2, please add), at the location of Gene ID: 4925.

The gene full length of the murine NUCB1 gene is 1,379 bp (with an accession number of NM_001163662.2, please add), at the location of Gene ID: 18220.

The gene full length of the murine NUCB2 gene is 1,262 bp (with an accession number of NM_001130479.2, please add), at the location of Gene ID: 53322.

The similarities between human and murine NUCB1 and NUCB2 at an DNA level are 84% for the similarity between mouse and human NUCB1, and 83% for the similarity between mouse and human NUCB2, respectively. The similarities of protein sequences are 89% for the similarity between mouse and human NUCB1, and 87% for the similarity between mouse and human NUCB2, respectively. It should be understood that substitutions of nucleotides in codons are acceptable when encoding the same amino acid. It should also be understood that, when nucleotide substitutions result in conservative amino acid substitutions, nucleotide changes are also acceptable.

Where an amino acid fragment of the NUCB is obtained, the nucleic acid sequence encoding it can be constructed according to it, and specific probes can be designed based on the nucleotide sequence. The full-length nucleotide sequence or fragments thereof can usually be obtained by PCR amplification, recombination or artificial synthesis. For PCR amplification, primers can be designed according to the NUCB nucleotide sequence disclosed in the present invention, especially the open reading frame sequence, and a commercially available cDNA library, or a cDNA library prepared according to conventional methods known to those skilled in the art can be used as a template to amplify the relevant sequence. When the sequence is long, two or more times of PCR amplification are often required, and then the fragments amplified each time are spliced together in a correct order.

Once the relevant sequences are obtained, they can be obtained in large quantities by a recombination method. This is usually to clone it into a vector, then transfer the vector into a cell, and then separate the relevant sequences from the proliferated host cells by conventional methods.

Moreover, the relevant sequences can also be synthesized artificially, especially when the fragment length is relatively short. Generally, a fragment with a very long sequence can be obtained by synthesizing several small fragments first and then connecting them.

Currently, the DNA sequence encoding the protein of the present invention (or the fragments, derivatives thereof) can be obtained entirely through chemical synthesis. This DNA sequence can then be introduced into a variety of existing DNA molecules (e.g. vectors) and cells known in the art.

The polynucleotide sequences of the present invention can be utilized to express or produce recombinant NUCB polypeptides through conventional recombinant DNA techniques. Generally speaking, there are the following steps:
(1) transforming or transducing a suitable host cell with the polynucleotide (or variant) encoding the human NUCB polypeptide of the present invention, or with a recombinant expression vector containing the polynucleotide;
(2) culturing the host cell in a suitable culture medium; and
(3) isolating and purifying proteins from the culture medium or the cells.

In the present invention, the NUCB polynucleotide sequence can be inserted into a recombinant expression vector. In summary, any plasmid or vector can be used, as long as it can replicate and be stabilized in the host. An important feature of an expression vector is that it usually contains an origin of replication, a promoter, a marker gene, and a translation control element.

Methods well known to those skilled in the art can be used for constructing an expression vector containing an NUCB encoding DNA sequence and an appropriate transcriptional/translational control signal. These methods include *in vitro* recombinant DNA technology, DNA synthesis technology, *in vivo* recombination technology, etc. The DNA sequence can be operably linked to an appropriate promoter in an expression vector to direct mRNA synthesis. The expression vector also includes a ribosome binding site for translation initiation and a transcription terminator.

Moreover, the expression vector preferably contains one or more selectable marker genes to provide a phenotypic trait for selection of transformed host cells, e.g. dihydrofolate reductase, neomycin resistance, and green fluorescent protein (GFP) for eukaryotic cell culture, or tetracycline or ampicillin resistance for *Escherichia coli.*

A vector containing the aforementioned appropriate DNA sequence and an appropriate promoter or control sequence can be used for transforming an appropriate host cell to enable it to express the protein.

The host cell can be a prokaryotic cell, such as a bacterial cell; or a lower eukaryotic cell, such as a yeast cell; or a higher eukaryotic cell, such as a mammalian cell. Representative examples include: bacterial cells of *Escherichia coli* and *Streptomyces;* fungal cells such as yeast; plant cells; insect cells; animal cells, etc.

Transformation of host cells with a recombinant DNA can be carried out using conventional techniques well known to those skilled in the art. When the host is a prokaryote such as *Escherichia coli,* competent cells that can absorb the DNA can be harvested after the exponential growth period, and treated by a CaCl₂ method, and the steps as used are well known in the field. Another method is to use MgC₂. If desired, transformation can also be performed by electroporation. When the host is a eukaryotic organism, the following DNA transfection methods can be used: a calcium phosphate co-precipitation method, a conventional mechanical method e.g. microinjection, electroporation, liposome packaging, etc.

The obtained transformants can be cultured using conventional methods to express the polypeptide encoded by the gene of the present invention. The medium used in the culture can be selected from various conventional media depending on the host cell as used. The cells are cultured under conditions suitable for the growth of the host cell. When the host cell has grown to an appropriate cell density, the selected promoter is induced by a suitable method (e.g. temperature conversion or chemical induction), and the cells are cultured for an additional period of time.

The recombinant polypeptide in the aforementioned method can be expressed intracellularly, on the cell membrane, or secreted extracellularly. If necessary, the recombinant protein can be isolated and purified by various separation methods utilizing its physical, chemical and other properties. These methods are well known to those skilled in the art. Examples of these methods include, but are not limited to, conventional renaturation treatment, treatment with a protein precipitant (salting-out method), centrifugation, osmotic shock, ultra-treatment, ultracentrifugation, molecular sieve chromatography (gel filtration), adsorption chromatography, ion exchange chromatography, high performance liquid chromatography (HPLC), and various other liquid chromatography techniques, and a combination of these methods.

In a preferred embodiment, the sequence of the NUCB1 protein is as shown in any one of SEQ ID NOs.1-2:

MPTSVPRGAPFLLLPPLLMLSAVLAVPVDRAAPPQEDSQATETPDTGLYYHRY
LQEVINVLETDGHFREKLQAANAEDIKSGKLSQELDFVSHNVRTKLDELKRQEVSRLRM
LLKAKMDAKQEPNLQVDHMNLLKQFEHLDPQNQHTFEARDLELLIQTATRDLAQYDA
AHHEEFKRYEMLKEHERRRYLESLGEEQRKEAERKLQEQQRRHREHPKVNVPGSQAQL
KEVWEELDGLDPNRFNPKTFFILHDINSDGVLDEQELEALFTKELEKVYDPKNEEDDMR
EMEEERLRMREHVMKNVDTNQDRLVTLEEFLASTQRKEFGDTGEGWKTVEMSPAYTE
EELKRFEEELAAREAELNARAQRLSQETEALGRSQDRLEAQKRELQQAVLQMEQRKQQ
LQEQSAPPSKPDGQLQFRADTDDAPVPAPAGDQKDVPASEKKVPEQPPELPQLDSQHL(
mouse NUCB1, SEQ ID NO.1)

MPPSGPRGTLLLLPLLLLLLLRAVLAVPLERGAPNKEETPATESPDTGLYYHRY
LQEVIDVLETDGHFREKLQAANAEDIKSGKLSRELDFVSHHVRTKLDELKRQEVSRLRM
LLKAKMDAEQDPNVQVDHLNLLKQFEHLDPQNQHTFEARDLELLIQTATRDLAQYDA
AHHEEFKRYEMLKEHERRRYLESLGEEQRKEAERKLEEQQRRHREHPKVNVPGSQAQL
KEVWEELDGLDPNRFNPKTFFILHDINSDGVLDEQELEALFTKELEKVYDPKNEEDDMR
EMEEERLRMREHVMKNVDTNQDRLVTLEEFLASTQRKEFGDTGEGWETVEMHPAYTE
EELRRFEEELAAREAELNAKAQRLSQETEALGRSQGRLEAQKRELQQAVLHMEQRKQQ

QQQQQGHKAPAAHPEGQLKFHPDTDDVPVPAPAGDQKEVDTSEKKLLERLPEVEVPQH
L(human NUCB1,SEQ ID NO.2)

In a preferred embodiment, the sequence of the NUCB2 protein is as shown in any one of SEQ ID NOs.3-4:

MRWRIIQVQYCFLLVPCMLTALEAVPIDVDKTKVHNTEPVENARIEPPDTGLY
YDEYLKQVIEVLETDPHFREKLQKADIEEIRSGRLSQELDLVSHKVRTRLDELKRQEVGR
LRMLIKAKLDALQDTGMNHHLLLKQFEHLNHQNPNTFESRDLDMLIKAATADLEQYDR
TRHEEFKKYEMMKEHERREYLKTLSEEKRKEEESKFEEMKRKHEDHPKVNHPGSKDQL
KEVWEETDGLDPNDFDPKTFFKLHDVNNDGFLDEQELEALFTRELEKVYNPQNAEDDM
IEMEEERLRMREHVMSEIDNNKDRLVTLEEFLRATEKKEFLEPDSWETLDQQQLFTEDE
LKEYESIIAIQENELKKRAEELQKQKEDLQRQHDHLEAQKQEYHQAVQHLEQKKLQQGI
APSGPAGELKFEPHT (mouse NUCB2, SEQ ID NO.3)

MRWRTILLQYCFLLITCLLTALEAVPIDIDKTKVQNIHPVESAKIEPPDTGLYYD
EYLKQVIDVLETDKHFREKLQKADIEEIKSGRLSKELDLVSHHVRTKLDELKRQEVGRL
RMLIKAKLDSLQDIGMDHQALLKQFDHLNHLNPDKFESTDLDMLIKAATSDLEHYDKT
RHEEFKKYEMMKEHERREYLKTLNEEKRKEEESKFEEMKKKHENHPKVNHPGSKDQL
KEVWEETDGLDPNDFDPKTFFKLHDVNSDGFLDEQELEALFTKELEKVYDPKNEEDDM
VEMEEERLRMREHVMNEVDTNKDRLVTLEEFLKATEKKEFLEPDSWETLDQQQFFTEE
ELKEYENIIALQENELKKKADELQKQKEELQRQHDQLEAQKLEYHQVIQQMEQKKLQQ
GIPPSGPAGELKFEPHI (human NUCB2, SEQ ID NO.4)

Mouse NUCB1 protein encoding nucleotide sequence (>NM_001163662.2:78-1457) (SEQ ID NO:5)

ATGCCTACCTCGGTGCCCCGCGGGGCCCCTTTTCTTCTCCTACCACCTCTAC
TGATGCTGTCTGCTGTGCTGGCAGTGCCCGTGGACCGCGCAGCACCTCCTCAGGAGG
ACAGCCAGGCCACTGAGACCCCGGACACGGGCCTGTACTACCACCGGTACCTCCAG
GAGGTCATCAACGTGCTAGAGACAGATGGGCACTTCCGGGAGAAGCTGCAGGCTGC

CAACGCTGAGGACATTAAGAGTGGAAAGCTGAGCCAAGAGCTGGACTTCGTCAGCC
ACAACGTCCGAACCAAGCTGGATGAGCTCAAGCGACAGGAGGTGTCGAGGCTGCGG
ATGCTGCTCAAGGCCAAGATGGATGCAAAGCAGGAGCCCAACTTGCAGGTGGACCA
CATGAACCTCCTGAAGCAGTTTGAGCACCTGGACCCTCAGAACCAGCACACGTTTGA
GGCTCGGGACCTAGAGCTGCTGATCCAGACGGCCACCCGAGACCTCGCCCAGTATG
ACGCTGCACACCATGAAGAGTTCAAACGCTACGAGATGCTCAAGGAACATGAGAGG
AGACGATACCTGGAGTCTCTGGGAGAGGAGCAGCGGAAGGAGGCCGAGAGGAAGC
TCCAAGAGCAACAGCGCAGACACCGGGAACACCCCAAAGTCAATGTTCCTGGCAGC
CAAGCCCAGTTGAAGGAGGTGTGGGAGGAGTTGGATGGATTGGACCCCAACAGGTT
CAACCCCAAGACCTTCTTCATACTGCATGACATCAACAGTGATGGTGTCCTAGATGA
GCAAGAACTGGAAGCTCTCTTTACCAAGGAGCTGGAGAAGGTGTATGACCCGAAGA
ATGAGGAGGATGACATGAGAGAGATGGAGGAAGAACGGCTTCGCATGCGGGAGCA
CGTGATGAAGAATGTGGACACCAACCAGGACCGGCTTGTGACCCTGGAGGAGTTCC
TGGCATCCACCCAGAGGAAGGAGTTCGGGGACACTGGGGAGGGGTGGAAGACAGT
GGAAATGTCCCCAGCCTACACAGAGGAGGAGCTGAAGCGTTTTGAAGAGGAGCTGG
CTGCCCGGGAAGCTGAGCTGAATGCCAGGGCCCAGCGCCTCAGCCAGGAGACAGAG
GCCCTGGGGCGCTCCCAGGACCGCCTGGAGGCACAGAAGAGAGAGCTGCAGCAGG
CTGTTCTGCAGATGGAGCAGAGGAAGCAGCAACTGCAAGAACAGAGCGCTCCGCCT
TCCAAACCTGACGGGCAGCTGCAGTTCCGTGCAGACACAGATGACGCTCCTGTCCCA
GCTCCAGCAGGTGACCAGAAAGATGTGCCTGCTTCTGAGAAGAAGGTCCCAGAGCA
GCCCCCTGAGCTGCCACAGCTGGATTCCCAGCATTTATAA

Human NUCB1 protein encoding nucleotide sequence (>NM_006184.6:73-1458) (SEQ ID NO.6)

ATGCCTCCCTCTGGGCCCCGAGGAACCCTCCTTCTGTTGCCGCTGCTGCTGC
TGCTCCTGCTTCGCGCCGTGCTGGCTGTCCCCCTGGAGCGAGGGGCGCCCAACAAGG
AGGAGACCCCTGCGACTGAGAGTCCCGACACAGGCCTGTACTACCACCGGTACCTC

CAGGAGGTCATCGATGTACTGGAGACGGATGGGCATTTCCGAGAGAAGCTGCAGGC
TGCCAATGCGGAGGACATCAAGAGCGGGAAGCTGAGCCGAGAGCTGGACTTTGTCA
GCCACCACGTCCGCACCAAGCTGGATGAGCTCAAGCGACAGGAGGTGTCACGGCTG
CGGATGCTGCTCAAGGCCAAGATGGACGCCGAGCAGGATCCCAATGTACAGGTGGA
TCATCTGAATCTCCTGAAACAGTTTGAACACCTGGACCCTCAGAACCAGCATACATT
CGAGGCCCGCGACCTGGAGCTGCTGATCCAGACGGCCACCCGGGACCTTGCCCAGT
ACGACGCAGCCCATCATGAAGAGTTCAAGCGCTACGAGATGCTTAAGGAACACGAG
AGACGGCGTTATCTGGAGTCACTGGGAGAGGAGCAGAGAAAGGAGGCGGAGAGGA
AGCTGGAAGAGCAACAGCGCCGGCACCGCGAGCACCCTAAAGTCAACGTGCCTGGC
AGCCAAGCCCAGTTGAAGGAGGTGTGGGAGGAGCTGGATGGACTGGACCCCAACA
GGTTTAACCCCAAGACCTTCTTCATACTGCATGATATCAACAGTGATGGTGTCCTGG
ATGAGCAGGAGCTGGAGGCACTCTTCACCAAGGAGCTGGAGAAAGTGTACGACCCA
AAGAATGAGGAGGACGACATGCGGGAGATGGAGGAGCGACTGCGCATGCGGG
AGCATGTGATGAAGAATGTGGACACCAACCAGGACCGCCTCGTGACCCTGGAGGAG
TTCCTCGCATCCACTCAGAGGAAGGAGTTTGGGGACACCGGGGAGGGCTGGGAGAC
AGTGGAGATGCACCCTGCCTACACCGAGGAAGAGCTGAGGCGCTTTGAAGAGGAGC
TGGCTGCCCGGGAGGCAGAGCTGAATGCCAAGGCCCAGCGCCTCAGCCAGGAGACA
GAGGCTCTAGGGCGGTCCCAGGGCCGCCTGGAGGCCCAGAAGAGAGAGCTGCAGC
AGGCTGTGCTGCACATGGAGCAGCGGAAGCAGCAGCAGCAGCAGCAAGGCCA
CAAGGCCCCGGCTGCCCACCCTGAGGGGCAGCTCAAGTTCCACCCAGACACAGACG
ATGTACCTGTCCCAGCTCCAGCCGGTGACCAGAAGGAGGTGGACACTTCAGAAAAG
AAACTTCTCGAGCGGCTCCCTGAGGTTGAGGTGCCCCAGCATCTGTGA

Mouse NUCB2 protein encoding nucleotide sequence (>NM_001130479.2:288-1550) (SEQ ID NO:7)

ATGAGGTGGAGGATCATCCAAGTACAGTACTGTTTTCTCTTGGTTCCGTGCA
TGCTGACCGCTCTGGAAGCTGTTCCTATCGATGTGGACAAGACCAAAGTACACAAC

ACTGAGCCAGTGGAAAATGCAAGGATAGAGCCACCAGATACTGGACTTTATTATGA
TGAATACCTCAAGCAAGTGATTGAAGTCTTGGAAACAGATCCACATTTCAGAGAAA
AGCTCCAGAAAGCAGACATAGAGGAGATAAGGAGCGGGAGGCTGAGTCAAGAGCT
GGACTTAGTAAGTCACAAAGTGAGGACGAGACTGGATGAGCTGAAGAGGCAAGAA
GTAGGAAGACTGCGGATGCTCATCAAAGCTAAGCTGGATGCCCTTCAAGACACTGG
CATGAATCACCACCTTCTTCTGAAGCAGTTTGAACACCTGAACCACCAGAATCCTAA
CACATTTGAATCCAGAGATTTGGATATGCTAATCAAAGCAGCTACCGCGGATCTGGA
GCAATATGACCGGACTCGGCATGAAGAGTTTAAGAAGTACGAGATGATGAAGGAAC
ACGAGCGGAGAGAGTATTTAAAAACGCTGAGTGAGGAGAAGAGGAAAGAAGAAGA
GTCTAAGTTTGAAGAGATGAAGAGGAAGCACGAAGACCACCCCAAAGTTAATCATC
CCGGAAGCAAAGATCAACTAAAAGAGGTTTGGGAAGAGACTGATGGATTGGACCCT
AATGACTTTGACCCCAAGACATTTTTCAAATTACATGATGTTAACAACGATGGATTC
CTGGATGAACAAGAATTAGAAGCACTATTCACAAGAGAGTTGGAGAAAGTGTATAA
CCCACAAAATGCAGAGGACGATATGATAGAAATGGAAGAGGAGAGGCTCAGGATG
AGAGAACACGTCATGAGTGAGATTGATAACAACAAAGACCGATTGGTGACTCTGGA
GGAATTCCTGAGAGCTACAGAGAAGAAAGAATTCCTGGAGCCTGATAGCTGGGAGA
CACTGGACCAGCAACAGTTATTCACCGAGGACGAGCTTAAAGAGTATGAAAGCATT
ATTGCTATCCAAGAGAACGAGCTTAAGAAGAGGGCGGAAGAGCTGCAGAAACAGA
AGGAGGATCTGCAGCGGCAGCACGACCACCTCGAGGCGCAGAAGCAGGAGTATCAT
CAGGCCGTCCAGCACCTGGAACAGAAGAAACTTCAACAAGGCATTGCTCCATCAGG
GCCAGCGGGAGAGCTGAAGTTTGAGCCACACACATAA

Human NUCB2 protein encoding nucleotide sequence (>NM_001352661.2:453-1718) (SEQ ID NO.8)

ATGATGAGGTGGAGGACCATCCTGCTACAGTATTGCTTTCTCTTGATTACAT
GTTTACTTACTGCTCTTGAAGCTGTGCCTATTGACATAGACAAGACAAAAGTACAAA
ATATTCACCCTGTGGAAAGTGCGAAGATAGAACCACCAGATACTGGACTTTATTATG

ATGAATATCTCAAGCAAGTGATTGATGTGCTGGAAACAGATAAACACTTCAGAGAA
AAGCTCCAGAAAGCAGACATAGAGGAAATAAAGAGTGGGAGGCTAAGCAAAGAAC
TGGATTTAGTAAGTCACCATGTGAGGACAAAACTTGATGAACTGAAAAGGCAAGAA
GTAGGAAGGTTAAGAATGTTAATTAAAGCTAAGTTGGATTCCCTTCAAGATATAGGC
ATGGACCACCAAGCTCTTCTAAAACAATTTGATCACCTAAACCACCTGAATCCTGAC
AAGTTTGAATCCACAGATTTAGATATGCTAATCAAAGCGGCAACAAGTGATCTGGA
ACACTATGACAAGACTCGTCATGAAGAATTTAAAAAATATGAAATGATGAAGGAAC
ATGAAAGGAGAGAATATTTAAAAACATTGAATGAAGAAAAGAGAAAAGAAGAAGA
GTCTAAATTTGAAGAAATGAAGAAAAAGCATGAAAATCACCCTAAAGTTAATCACC
CAGGAAGCAAAGATCAACTAAAAGAGGTATGGGAAGAGACTGATGGATTGGATCCT
AATGACTTTGACCCCAAGACATTTTTCAAATTACATGATGTCAATAGTGATGGATTC
CTGGATGAACAAGAATTAGAAGCCCTATTTACTAAAGAGTTGGAGAAAGTATATGA
CCCTAAAAATGAAGAGGATGATATGGTAGAAATGGAAGAAGAAAGGCTTAGAATG
AGGGAACATGTAATGAATGAGGTTGATACTAACAAAGACAGATTGGTGACTCTGGA
GGAGTTTTTGAAAGCCACAGAAAAAAAAGAATTCTTGGAGCCAGATAGCTGGGAGA
CATTAGATCAGCAACAGTTCTTCACAGAGGAAGAACTAAAAGAATATGAAAATATT
ATTGCTTTACAAGAAAATGAACTTAAGAAGAAGGCAGATGAGCTTCAGAAACAAAA
AGAAGAGCTACAACGTCAGCATGATCAACTGGAGGCTCAGAAGCTGGAATATCATC
AGGTCATACAGCAGATGGAACAAAAAAAATTACAACAAGGAATTCCTCCATCAGGG
CCAGCTGGAGAATTGAAGTTTGAGCCACACATTTAA

### Adeno-associated virus

Because the adeno-associated virus (AAV) is smaller than other viral vectors, has no pathogenicity, and can transfect both dividing and non-dividing cells, etc., gene therapy methods based on AAV vectors against genetic diseases have attracted extensive attention.

The adeno-associated virus (AAV), also known as adeno associated virus, belongs to the genus *Dependovirus* in the family *Parvoviridae.* It is a kind of single-stranded DNA-deficient virus with the simplest structure found currently, and needs a helper virus (usually adenovirus) to participate in replication. It encodes the cap and rep genes within two inverted terminal repeats (ITRs). The ITRs play a decisive role in viral replication and packaging. The cap gene encodes the viral capsid protein, and the rep gene is involved in viral replication and integration. AAV can infect a variety types of cells.

Recombinant adeno-associated virus (rAAV) vectors are derived from non-pathogenic wild-type adeno-associated viruses. Due to their good safety, wide range of host cells (dividing and non-dividing cells), low immunogenicity, long-term expression of exogenous genes *in vivo,* and the like features, they are considered as one of the most promising gene transfer vectors and are widely used in gene therapy and vaccine research worldwide. After more than 10 years of research, the biological characteristics of recombinant adeno-associated viruses have been deeply understood, especially a lot of data have been accumulated on their application effects in various cells, tissues and *in vivo* experiments. In medical research, rAAV is used in gene therapy studies for a variety of diseases (including *in vivo* and *in vitro* experiments); at the same time, as an unique characteristic gene transfer vector, it is also widely used in aspects such as gene function research, construction of disease models, and preparation of gene knockout murine.

In a preferred embodiment of the present invention, the vector is a recombinant AAV vector. AAVs are relatively small DNA viruses that can integrate into the genome of the cells they infect in a stable and site-specific manner. They are able to infect a wide range of cells without any effects on cell growth, morphology or differentiation, and they do not appear to be involved in human pathology. The AAV genome has been cloned, sequenced, and characterized. The AAV contains an inverted terminal repeat (ITR) region of approximately 145 bases at each end that serves as the viral origin of replication. The rest of the genome is divided into two important regions with encapsidation functions: the left part of the genome containing the rep genes involved in viral replication and viral gene expression; and the right part of the genome containing the cap gene encoding the viral capsid protein.

AAV vectors can be prepared using standard methods in the art. Any serotype of adeno-associated virus is suitable. Methods for purifying vectors can be found in, for example, US Patent Nos. 6566118, 6989264, and 6995006, the disclosures of which are incorporated herein by reference in their entireties. Preparation of hybrid vectors is described, for example, in PCT Application No. PCT/US2005/027091, the disclosure of which is incorporated herein by reference in its entirety. The use of AAV-derived vectors for *in vitro and in vivo* gene transfer has been described (referring to, e.g., International Patent Application Publication Nos. WO91/18088 and WO93/09239; US Patent Nos. 4,797,368, 6,596,535, and 5,139,941, and European Patent No. 0488528, all of which are incorporated herein by reference in their entireties). These patent publications describe various AAV-derived constructs in which the rep and/or cap genes are deleted and replaced with a gene of interest, and the use of these constructs to deliver the gene of interest *in vitro* (into cultured cells) or *in vivo* (directly into an organism). A replication-defective recombinant AAV can be prepared by co-transfecting a cell line infected with a human helper virus (e.g. adenovirus) with a plasmid containing the nucleic acid sequence of interest flanked by two AAV inverted terminal repeat (ITR) regions and a plasmid carrying the AAV encapsidation genes (rep and cap genes). The resulting AAV recombinants are then purified by standard techniques.

In some embodiments, the recombinant vector is encapsidated into a viral particle (e.g. an AAV viral particle including but not limited to AAV1, AAV2, AAV3, AAV4, AAV5, AAV6, AAV7, AAV8, AAV9, AAV10, AAV11, AAV12, AAV13, AAV14, AAV15, and AAV16). Thus, the present disclosure includes recombinant viral particles (recombinant in that they contain a recombinant polynucleotide) containing any of the vectors described herein. Methods for producing such particles are known in the art and are described in US Patent No. 6,596,535.

### Pharmaceutical composition

The present invention further provides a pharmaceutical composition including (a) the NUCB protein, or an encoding gene or expression vector thereof of the present invention; and (b) a safe and effective pharmaceutically acceptable carrier. The severity of the disease in the patient to be treated, the patient's body weight, the patient's immune status, the route of administration, etc. Generally, satisfactory effects can be obtained when the active ingredient of the present invention is administered at a dose of about 0.00001 mg-50 mg/kg body weight of the animal (preferably 0.0001 mg-25 mg/kg body weight of the animal) per day (the dosage for the experimental mice is 25 mg/kg). For example, several divided doses may be administered daily or the dose may be proportionally reduced as indicated by the exigencies of the therapeutic situation. Moreover, the NUCB protein, or an encoding gene or expression vector thereof of the present invention can be used alone or together with other therapeutic agents (e.g. formulated in the same pharmaceutical composition).

The pharmaceutical composition may further contain a pharmaceutically acceptable carrier. The term "pharmaceutically acceptable carrier" refers to a carrier used for administering a therapeutic agent. The term refers to such pharmaceutical carriers that do not themselves induce the production of antibodies harmful to the individual receiving the composition and that are not unduly toxic upon administration. These vectors are well known to those skilled in the art. A thorough discussion of pharmaceutically acceptable excipients can be found in Remington's Pharmaceutical Sciences (Mack Pub. Co., N.J.1991). Such carriers include (but are not limited to): saline, a buffer, dextrose, water, glycerol, ethanol, an adjuvant, and a combination thereof.

The pharmaceutically acceptable carrier in the therapeutic composition may contain a liquid such as water, saline, glycerol, and ethanol. Moreover, these carriers may also contain auxiliary substances, such as wetting agents or emulsifiers, pH buffering substances, etc.

Typically, the therapeutic composition can be prepared into an injectable, either as a liquid solution or suspension; and can also be prepared into a solid form suitable for formulating into a solution or suspension, or a liquid vehicle prior to injection.

Once formulated, the composition of the present invention can be administered by conventional routes, including (but not limited to): intratumoral, intramuscular, intravenous, subcutaneous, intradermal, or topical administration. The subject to be prevented or treated may be an animal; and in particular, human.

When the pharmaceutical composition of the present invention is used for actual treatment, various dosage forms of the pharmaceutical composition can be adopted according to the usage. Preferably, it is an intravenous preparation.

These pharmaceutical compositions can be formulated according to conventional methods by mixing, diluting or dissolving, and occasionally added with suitable pharmaceutical additives such as excipients, disintegrants, binders, lubricants, diluents, buffers, isotonicities, preservatives, wetting agents, emulsifiers, dispersants, stabilizers and solubilizers, and the formulation process can be carried out in a conventional manner according to the dosage form.

For example, eye drops can be prepared as follows: dissolving the NUCB protein, or an encoding gene or expression vector thereof of the present invention together with the basic substances in sterile water (with a surfactant dissolved in the sterile water), adjusting the osmotic pressure and pH to physiological conditions, and optionally adding suitable pharmaceutical additives such as preservatives, stabilizers, buffers, isotonic agents, antioxidants and viscosity enhancers, and then completely dissolving them.

The pharmaceutical composition of the present invention can also be administered in the form of a sustained-release formulation. For example, the NUCB protein, or an encoding gene or expression vector thereof of the present invention can be incorporated into a pill or microcapsule with a slow-release polymer as a carrier, and then the pill or microcapsule is surgically implanted into a tissue to be treated. Examples of the sustained-release polymer include a ethylene-vinyl acetate copolymer, polyhydroxymethacrylate, polyacrylamide, polyvinylpyrrolidone, methylcellulose, a lactic acid polymer, a lactic acid-glycolic acid copolymer, etc., and preferably, a biodegradable polymer e.g. a lactic acid polymer and a lactic acid-glycolic acid copolymer.

When the pharmaceutical composition of the present invention is used for actual treatment, the dosage of the NUCB protein, or an encoding gene or expression vector thereof of the present invention as the active ingredient can be reasonably determined according to the body weight, age, sex, and symptom severity of each patient to be treated.

The method of the present invention can ameliorate or treat (a) body weight reduction and (b) body fat reduction caused by cachexia, thereby improving the quality of life of patients.

### The main advantages of the present invention include:

(1) The present invention unexpectedly discovered for the first time that nucleobindins (NUCBs) can ameliorate or treat cachexia. Furthermore, it has been demonstrated through experiments that administering NUCB proteins, such as NUCB1 and/or NUCB2 proteins, to a cachexia mouse model can significantly ameliorate cachexia-induced (a) body weight reduction and (b) body fat reduction.
(2) The present invention has discovered for the first time that cachexia can be effectively treated or ameliorated by exogenous low-dose expression of NUCB, especially NUCB 1 and/or NUCB2.
(3) The method of the present invention is simple to operate, simple to prepare the drug, and highly efficient.

The present invention will be further described hereafter in connection with specific examples. It should be understood that, the following examples are only intended to illustrate the present invention, rather than limiting the scope of the present invention. The experimental methods in the following examples which are not specified with specific conditions are generally carried out according to conventional conditions, such as those described in Sambrook et al., Molecular Cloning: Laboratory Manual (New York: Cold Spring Harbor Laboratory Press, 1989), or those recommended by the manufacturer . Unless otherwise stated, percentages and parts are weight percentages and parts by weight. Unless otherwise specified, the materials and reagents used in the examples of the present invention are commercially available products.

In the present invention, mouse experiments all use mouse gene sequences and express mouse NUCB1-2 proteins.

### General Methods

### 1. AAV virus and tail vein injection

AAV2/8-Control virus (pAAV-CAG-MCS-3 × FLAG-WPRE), AAV2/8-NUCB1 virus (pAAV-CAG-NUCB1-3 × FLAG-WPRE), and AAV2/8-NUCB2 virus (pAAV-CAG-NUCB2-3 × FLAG-WPRE) (purchased from OBiO Technology). All 3 AAV viruses are administered by tail vein injection. Each mouse (mouse name C57BL/6J, purchased from Shanghai Lingchang Bio-tech Company Limited) is given 1 × 10¹⁰ genome copies of AAV viruses, which are dissolved in 100 ul of physiological saline.

### 2. Western Blot

A 10% SDS-PAGE gel is prepared and added with a protein sample for separation by electrophoresis. The protein in the gel is transferred to an NC (nitrocellulose) membrane, and blocked with 0.5% skim milk for 1 hour. The sections are washed with TBST for 3 times, with 5 minutes each time, then added with the corresponding primary antibody (rabbit-derived anti-FLAG antibody, CST), and incubated at 4°C overnight. After antibody recovery, the membrane is washed for 3 times with TBST, with 5 minutes each time, added with a corresponding HRP-conjugated secondary antibody (Sigma), and incubated at room temperature for 1 hour. After washing for three times, a developer solution is added for scanning.

### 3. Cell Culture

LLC (Lewis Lung cancer) cells (purchased from the Cell Bank of the Chinese Academy of Sciences) are cultured in a DMEM (Invitrogen) medium containing 10% fetal bovine serum at 37°C and a CO₂ concentration of 5%. Before inoculation of the mice, the digested cells are washed with PBS for 3 times, filtered through a 0.45 uM filter sieve, and dissolved in PBS at a concentration of 1 * 10⁷/m1. Each mouse is inoculated subcutaneously with 100 ul of the solution.

### 4. Cachexia Mouse Model

C57BL/6 mice are purchased from the Lingchang Company and raised in an SPF-level animal room with a normal diet (13.5% kcal), with lights off for 12 hours (19:30-07:30) and lights on for 12 hours (07:30-19:30). In this study, cachexia is induced in the mice by inoculating Lewis lung cancer cells, which is the most commonly used method to construct a cachexia mouse model. Specifically: the experimental mice are randomly divided into 4 groups (a non-LLC inoculation group, an LLC inoculation and control virus injection group, an LLC inoculation and NUCB1 virus injection group, and an LLC inoculation and NUCB2 virus injection group). On day 0, the body weight of each mouse is weighed, and each mouse in the LLC inoculation group is subcutaneously inoculated with 1 * 10⁶ LLC cells. On day 1, the mice in the LLC inoculation group are given 1 × 10¹⁰ genome copies of the corresponding AAV virus by tail vein injection. Subsequently, the tumor size (calculated as: volume = length * width * width / 2) and the food intake of the mice are measured every week. On day 30, the mice are sacrificed, and the tumor weight, the weight of the mice, and the weight of epididymal white adipose tissues are weighed. A small amount of liver tissues is taken to detect the expression of the AAV virus.

### 5. Data Statistical Analysis

The data are presented as means ± standard error (means ± s.e.m). P values are obtained by a t test (student's test) or one-way ANOVA, and significance is expressed as *: P < 0.05, **: P < 0.01, ***: P < 0.001.

### Example 1: Well expression of NUCB1 and NUCB2 proteins administered by AAV virus in mice

As shown in FIG. 1A, the experimental process was to inoculate LLC cells on day 0, administer an AAV virus on the first day, and sample for detect on day 30. The liver was an important site of expression of the serotype AAV2/8 virus. On day 30, the liver was removed to detect the expression of NUCB1 and NUCB2. As shown in FIG. 1B, the FLAG tags of NUCB1 and NUCB2 were detected by Western Blot, indicating that AAV-overexpressed NUCB1 and NUCB2 were well expressed in the mice.

### Example 2 NUCB1 and NUCB2 proteins administered by AAV virus significantly ameliorate cachexia symptoms in mice

As shown in FIGs. 2A and 2B, expression of NUCB1 and NUCB2 did not alter LLC tumor growth, as evidenced by no difference in tumor size and final tumor weight. FIGs. 2C and 2D showed two points. First, the mouse cachexia model was successful, as shown by the increase in the body weights of the mice not inoculated with LLC cells and the significant reduction in the body weights of the mice inoculated with LLC cells and receiving the control virus. Second, overexpression of NUCB1 and NUCB2 through the AAV virus could significantly improve the body weight reduction of the mice. As shown in FIG. 2E, the cachexia-induced decrease in body fat (epididymal white fat) in the mice was also well ameliorated by the overexpression of NUCB1 and NUCB2. Decreased appetite was another important manifestation of cachexia. As shown in FIG. 2F, the overexpression of NUCB1 and NUCB2 did not ameliorate the reduced food intake caused by cachexia.

### Discussion

NUCB proteins are a family of calcium ion-binding secretory proteins, including NUCB1 and NUCB2. The function of the NUCB2 protein was first reported in 2006 to suppress appetite and reduce body weight. It has been found by subsequent studies that under physiological conditions, the NUCB2 protein does not affect the body weight and food intake of the mice. Therefore, the biological function and mechanism of the NUCB family, namely NUCB1 and NUCB2 proteins, are still unclear. The present invention is the first to discover that nucleobindin (NUCB) can effectively ameliorate or treat cachexia. In a mouse model of cachexia, the NUCB1 and NUCB2 proteins treat and ameliorate the quality of life of patients with cachexia by (a) increasing the body weight and (b) increasing the body fat. At the same time, it also provides new possibilities for screening a novel method to ameliorate or treat cachexia.

All documents mentioned in the present invention are incorporated by reference into the present application as if each document is individually incorporated by reference. Furthermore, it should be understood that after reading the aforementioned content of the present invention, those skilled in the art can make various changes or modifications to the present invention, and these equivalent forms also fall within the scope defined by the appended claims of the present application.

## Claims

1. Use of nucleobindin (NUCBs), or an encoding gene or expression vector thereof, for preparing a composition or formulation for ameliorating or treating cachexia.

2. The use according to claim 1, wherein the cachexia comprises tumor cachexia.

3. The use according to claim 2, wherein the tumor comprises lung cancer, pancreatic cancer, gastroesophageal cancer, head and neck cancer, colorectal cancer, blood cancer, breast cancer, and prostate cancer.

4. The use according to claim 1, wherein the NUCB protein is selected from the group consisting of NUCB1, NUCB2, or a combination thereof.

5. The use according to claim 1, wherein the composition or formulation further comprises other drugs for ameliorating or treating cachexia.

6. A pharmaceutical composition, comprising:
(a1) a first active ingredient for ameliorating or treating cachexia, the first active ingredient comprising: an NUCB protein, or an encoding gene or expression vector thereof;
(a2) optionally, a second active ingredient for ameliorating or treating cachexia, the second active ingredient comprising: other drugs for ameliorating or treating cachexia; and
(b) a pharmaceutically acceptable carrier.

7. The pharmaceutical composition according to claim 6, wherein the other drugs for ameliorating or treating cachexia are selected from the group consisting of a corticosteroid, a progesterone analog, olanzapine, anamorelin, or a combination thereof.

8. A kit, comprising:
(i) a first container, and an active ingredient located in the first container: (a1) NUCB protein, or an encoding gene or expression vector thereof, or a drug containing an active ingredient (a); and
(ii) optionally a second container, and an active ingredient located in the second container: (a2) other drugs for ameliorating or treating cachexia, or a drug containing the active ingredient (a2).

9. Use of the pharmaceutical composition according to claim 6 or the kit according to claim 8 in preparation of a drug for ameliorating or treating cachexia.

10. A method for screening a candidate drug for ameliorating or treating cachexia, comprising the steps of:
(a) in a culture system of in a test group in the presence of a test substance, culturing cells expressing an NUCB protein for a period of time T1, and detecting an expression level E1 and/or activity A1 of the NUCB protein in the culture system of the test group;
and in a control group in the absence of the test substance and with the same other conditions, detecting an expression level E2 and/or activity A2 of the NUCB protein in the culture system of the control group;
(bl) comparing E1 and E2, wherein if E1 is significantly higher than E2, it indicates that the test substance is a candidate drug for ameliorating or treating cachexia; and/or
(b2) comparing A1 and A2, wherein if A1 is significantly higher than A2, it indicates that the test substance is a candidate drug for ameliorating or treating cachexia.

11. A method for screening a candidate drug for treating or ameliorating cachexia, comprising the steps of:
(a) in a test group in the presence of a test compound, detecting an mRNA expression level or protein level L1 of NUCB1 and/or NUCB2 genes in cells of the test group;
and in a control group in the absence of the test compound and with the same other conditions, detecting an mRNA expression level or protein level L2 of the NUCB1 and/or NUCB2 in the control group; and
(b) comparing L1 and L2, wherein if L2 is significantly lower than L1, it indicates that the test compound is a candidate drug for treating or ameliorating cachexia.
